# DEMANDE DE BREVET EUROPEEN

(11) **EP 2 008 647 A1**
(43) Date de publication de la demande: **31.12.2008**
(21) Numéro de dépôt: 08158201.7
(22) Date de dépôt: 13.06.2008
(51) Int. Cl.: A61K 8/85, A61Q 1/06, C08G 63/48

(54) **Composition cosmétique ou pharmaceutique comprenant un polycondensat, procédé de traitement cosmétique, polycondensat et procédé de préparation**

(30) Priorité: 04.10.2007 FR 0758059; 21.06.2007 FR 0755919
(71) Demandeur: L'Oreal, 75008 Paris (FR)
(72) Inventeur: Giustianiani, Pascal, 92250, La Garenne Colombes (FR); Malle, Gérard, 77580, Villiers s/Morin (FR)
(74) Mandataire: Dodin, Catherine

(57) **Abrégé**

La présente demande concerne une composition cosmétique ou pharmaceutique, notamment de rouge à lèvres, comprenant un polycondensat susceptible d'être obtenu par réaction :
- de polyol comprenant 3 à 6 groupes hydroxyles;
- d'un mélange d'acides monocarboxyliques non aromatiques, les uns de température de fusion supérieure à 25°C, les autres de température de fusion inférieure à 25°C,
- d'acide monocarboxylique aromatique comprenant 7 à 11 atomes de carbone; et
- d'acide polycarboxylique, saturé ou insaturé, voire aromatique, linéaire, ramifié et/ou cyclique, comprenant au moins 2 groupes carboxyliques COOH et pouvant comprendre des hétéroatomes; et/ou un anhydride cyclique d'un tel acide polycarboxylique et/ou d'une lactone.

La demande concerne également un procédé de traitement cosmétique employant ladite composition, le polycondensat ainsi défini et un procédé de préparation dudit polycondensat.

## Description

La présente invention a trait à de nouveaux polymères de la famille des polycondensats de type alkyde modifié, ainsi qu'à leur utilisation dans les compositions cosmétiques, notamment dans les rouges à lèvres, aux compositions cosmétiques les contenant et aux procédés de préparation desdits polycondensats.

Il existe de nombreuses compositions cosmétiques pour lesquelles des propriétés de brillance du film déposé, après application sur les matières kératiniques (peau, lèvres, phanères), sont souhaitées. On peut citer par exemple les rouges à lèvres, les vernis à ongles ou encore certains produits capillaires.

Afin d'obtenir un tel résultat, il est possible d'associer des matières premières particulières, notamment des lanolines, avec des huiles dites brillantes, telles que les polybutènes qui présentent toutefois une viscosité élevée; ou des esters d'acide ou d'alcool gras dont le nombre de carbone est élevé; ou bien certaines huiles végétales; ou encore des esters résultants de l'estérification partielle ou totale d'un composé aliphatique hydroxylé avec un acide aromatique, comme décrit dans la demande de brevet EP1097699.

Il est également connu d'associer des lanolines avec des polyesters obtenus par réaction séquencée de l'huile de ricin avec l'acide isostéarique puis avec l'acide succinique, tel que décrit dans le brevet US6342527.

Pour améliorer la brillance du film déposé, ainsi que sa tenue, il a également été proposé d'utiliser des esters résultant de la condensation d'un polyol avec un acide carboxylique de type "néo", notamment dans FR2838049.

On peut également citer EP1457201, qui décrit une composition associant un polyester de triglycérides d'acides carboxyliques hydroxylés et une huile de faible masse moléculaire choisie parmi les polybutylènes, les polyisobutylènes hydrogénés, les polydécènes hydrogénés ou non, les copolymères de vinylpyrrolidones, les esters d'acides gras linéaires, les esters hydroxylés, les esters d'alcools gras ou d'acides gras ramifiés en C24-C28, les huiles siliconées et/ou les huiles d'origine végétale.

Dans la demande de brevet EP0792637, il est décrit une composition associant un ester aromatique et un polymère de type polybutène ou polyisobutène.

Dans la demande de brevet EP1155687, on décrit un procédé consistant à incorporer, dans une phase huileuse constituée d'une huile cosmétiquement acceptable, un organopolysiloxane possédant au moins 2 groupes susceptibles d'établir des liaisons hydrogènes.

Toutefois, ces compositions et associations, même si elles améliorent la brillance de manière significative, sont encore jugées insuffisantes dans une optique de longue tenue de cette brillance, dans le temps.

Le but de la présente invention est de proposer de nouveaux polymères qui peuvent conférer une brillance significative à un dépôt notamment filmogène, tout en maintenant une bonne durabilité dans le temps de cette brillance; ceci peut trouver une application particulièrement avantageuse dans le domaine des rouges à lèvres. Par ailleurs, on recherche également des polymères peuvent en outre avantageusement conférer à la composition une excellente tenue dans le temps sur les matières kératiniques, notamment sur les lèvres.

A cette fin, la demanderesse a recherché de nouveaux polycondensats de type alkyde, ayant les propriétés recherchées.

Les résines alkydes constituent une classe particulière de polyesters en étant le produit de réaction de polyols et d'acides polycarboxyliques, généralement modifié par des acides gras insaturés, tels que l'acide oléique, ou par des huiles insaturées, huile de soja ou de ricin par exemple, qui permettent de moduler leurs propriétés filmogènes, notamment leur vitesse de séchage, leur dureté, leur résistance.

Ainsi, il a été proposé dans le document US2915488 des résines alkydes modifiées dans lesquelles une partie des acides gras provenant de l'huile de soja a été remplacée par de l'acide benzoïque. Ces nouvelles résines présentent des propriétés améliorées en terme de résistance aux alcalis et aux détergents; les films les contenant sèchent plus rapidement et sont plus durs. Toutefois, aucune application, notamment cosmétique ou topique, n'a été envisagée pour ces résines.

Par ailleurs, les acides gras présents dans l'huile de soja sont majoritairement constitués de deux acides gras insaturés: environ 55% d'acide linoléique (C18:2) et 28% d'acide oléique (C18:1), selon "Surface Coatings Science and Technology", 2ème édition, JOHN WILEY & Sons, pages 104 et 105. Or, on sait que certains acides gras insaturés peuvent subir, au cours du temps, une autooxydation qui peut être à l'origine de phénomènes de rancissement, qui peuvent déboucher sur des problèmes de conservation des compositions comprenant ces matières premières. De plus, les résines alkydes décrites dans US2915488, qui comprennent une proportion élevée d'acides gras linoléique et oléique, ne sont pas optimales, notamment en terme de stabilité, pour une utilisation en cosmétique.

La réticulation des résines alkydes par oxydation à l'air avec formation d'hydroperoxydes joue un rôle clé dans la vitesse de séchage du film, et, par suite, dans ses propriétés finales de dureté et de résistance aux agressions extérieures. Dans le domaines habituel d'application de ces résines alkydes, à savoir les peintures, la vitesse de réticulation, et donc de séchage, est généralement accélérée par addition de sels de métaux particuliers, dénommés "driers", tels que les naphténates et octanoates de cobalt qui accélèrent la décomposition des hydroperoxydes; ceci est notamment décrit dans Principles of Polymerization, 4ème édition, JOHN WILEY & SONS, pages 737-738 et également dans Surface Coatings Science and Technology, 2ème édition, JOHN WILEY & SONS, tables 2.3 et 2.4 pages 526-530.

Toutefois, l'utilisation de tels sels métalliques n'est bien évidemment pas souhaitable en cosmétique pour des raisons évidentes de toxicité.

Par ailleurs, la plupart des résines alkydes n'a pas une solubilité convenable dans les milieux huileux habituellement utilisés en cosmétique, tels que les huiles végétales, les alcanes, les esters gras, les alcools gras, les huiles de silicone, et notamment comprenant l'isododécane, le Parléam, l'isononanoate d'isononyle, l'octyldodécanol, la phényl triméthicone, le benzoate d'alkyle en C12-C15 et/ou la D5 (décaméthylcyclopentasiloxane).

Après d'importantes recherches, la demanderesse a découvert de façon surprenante et inattendue que certains polycondensats à plus haute teneur en acides carboxyliques particuliers, dont les acides monocarboxyliques non aromatiques, pouvaient conduire à des performances améliorées en terme de brillance, de maintien de ladite brillance, et en outre de longue tenue du film obtenu, tout en étant véhiculables dans les milieux cosmétiques usuels, notamment les milieux huileux cosmétiques usuels.

La présente invention a donc pour objet une composition cosmétique ou pharmaceutique comprenant, dans un milieu cosmétiquement ou pharmaceutiquement acceptable, au moins un polycondensat susceptible d'être obtenu par réaction :
- de 10 à 30% en poids, par rapport au poids total du polycondensat, d'au moins un polyol comprenant 3 à 6 groupes hydroxyles;
- de 22 à 80% en poids, par rapport au poids total du polycondensat, d'au moins un acide monocarboxylique non aromatique, saturé ou insaturé, linéaire, ramifié et/ou cyclique, comprenant 10 à 32 atomes de carbone, et ayant une température de fusion supérieure ou égale à 25°C;
- de 0,1 à 35% en poids, par rapport au poids total du polycondensat, d'au moins un acide monocarboxylique non aromatique, saturé ou insaturé, linéaire, ramifié et/ou cyclique, comprenant 6 à 32 atomes de carbone, et ayant une température de fusion strictement inférieure à 25°C;
- de 0,1 à 10 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 1 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, pouvant comprendre des hétéroatomes; et/ou un anhydride cyclique d'un tel acide polycarboxylique; et/ou une lactone comprenant au moins un groupement COOH.

Un autre objet de l'invention est un polycondensat susceptible d'être obtenu par réaction :
- de 10 à 30% en poids, par rapport au poids total du polycondensat, d'au moins un polyol comprenant 3 à 6 groupes hydroxyles;
- de 22 à 80% en poids, par rapport au poids total du polycondensat, d'au moins un acide monocarboxylique non aromatique, saturé ou insaturé, linéaire, ramifié et/ou cyclique, comprenant 10 à 32 atomes de carbone, et ayant une température de fusion supérieure ou égale à 25°C;
- de 0,1 à 35% en poids, par rapport au poids total du polycondensat, d'au moins un acide monocarboxylique non aromatique, saturé ou insaturé, linéaire, ramifié et/ou cyclique, comprenant 6 à 32 atomes de carbone, et ayant une température de fusion strictement inférieure à 25°C;
- de 0,1 à 10% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 1 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH, et pouvant comprendre un ou plusieurs hétéroatomes; et/ou un anhydride cyclique d'un tel acide polycarboxylique; et/ou une lactone comprenant au moins un groupement COOH.

Au final, le polycondensat comprend donc généralement ces monomères, dans les proportions indiquées; de manière très préférentielle, le polycondensat est constitué (comprend exclusivement ou uniquement) par ces monomères.

Notamment, les compositions cosmétiques présentent une bonne applicabilité et une bonne couvrance; une bonne adhérence sur le support, que cela soit sur l'ongle, les cheveux, les cils, la peau ou les lèvres; une flexibilité et une résistance du film adéquates, afin d'éviter les craquelures, par exemple dans le cas des vernis ou des rouges à lèvres; ainsi qu'un excellent niveau de brillance durable.

Les propriétés de confort et de glissant sont également très satisfaisantes.

Ces polycondensats sont aisément véhiculables dans les milieux solvants ou huileux cosmétiques, notamment les huiles, les alcools gras et/ou les esters gras, ce qui facilite leur mise en oeuvre dans le domaine cosmétique, notamment dans les rouges à lèvres ou les fonds de teint.

Les polycondensats selon l'invention peuvent être préparés aisément, en une seule étape de synthèse, et sans produire de déchets, ceci à faible coût.

Par ailleurs, il est aisément possible de modifier la structure et/ou les propriétés des polycondensats selon l'invention, en faisant varier la nature chimique des différents constituants et/ou leurs proportions.

Les polycondensats selon l'invention sont avantageusement branchés (ramifiés); on peut penser que ceci permet de générer un réseau par enchevêtrement des chaînes polymériques, et donc d'obtenir les propriétés recherchées, notamment en terme de tenue améliorée, de brillance améliorée, et en terme de solubilité. On a en effet constaté que les polycondensats linéaires ne permettaient pas d'obtenir une amélioration notable de la tenue de la composition, et que les polycondensats de type dendrimères, dont les chaînes sont régulières, ne présentaient pas une solubilité optimale.

Les polycondensats selon l'invention sont des polycondensats de type alkyde, et sont donc susceptibles d'être obtenus par estérification/polycondensation, selon les méthodes connues de l'homme du métier, des constituants décrits ci-après.

L'un des constituants nécessaires pour la préparation des polycondensats selon l'invention est un composé comprenant 3 à 6 groupes hydroxyles (polyol), notamment 3 à 4 groupes hydroxyles. On peut bien évidemment utiliser un mélange de tels polyols.

Ledit polyol peut notamment être un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther).

Ledit polyol est de préférence un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

Il peut être choisi parmi, seul ou en mélange :
- les triols, tels que le 1,2,4-butanetriol, le 1,2,6-hexanetriol, le triméthyloléthane, le triméthylolpropane, le glycérol;
- les tétraols, tels que le pentaérythritol (tétraméthylolméthane), l'érythritol, le diglycérol ou le ditriméthylolpropane;
- les pentols tels que le xylitol,
- les hexols tels que le sorbitol et le mannitol; ou encore le dipentaérythritol ou le triglycérol.

De préférence, le polyol est choisi parmi le glycérol, le diglycérol, le pentaérythritol, le sorbitol et leurs mélanges; et encore mieux est du pentaérythritol.

Le polyol, ou le mélange de polyol, représente de préférence 10 à 30% en poids, notamment 12 à 25% en poids, et mieux 14 à 22% en poids, du poids total du polycondensat final.

Un autre constituant nécessaire pour la préparation des polycondensats selon l'invention est un acide monocarboxylique non aromatique, saturé ou insaturé, linéaire, ramifié et/ou cyclique, comprenant 10 à 32 atomes de carbone, notamment 12 à 28 atomes de carbone et encore mieux 12 à 24 atomes de carbone; et ayant une température de fusion supérieure ou égale à 25°C, notamment supérieure ou égale à 28°C, voire à 30°C. On peut bien évidemment utiliser un mélange de tels acides monocarboxyliques non aromatiques.

On a constaté que lorsque l'on emploie un tel acide, dans les quantités indiquées, il est possible, d'une part d'obtenir une bonne brillance et la tenue de ladite brillance, et d'autre part de réduire la quantité de cires usuellement présentes dans la composition envisagée.

Par acide monocarboxylique non aromatique, on entend un composé de formule RCOOH, dans laquelle R est un radical hydrocarboné saturé ou insaturé, linéaire, ramifié et/ou cyclique, comprenant 9 à 31 atomes de carbone, notamment 11 à 27 atomes de carbone, et encore mieux 11 à 23 atomes de carbone.

De préférence, le radical R est saturé. Encore mieux, ledit radical R est linéaire ou ramifié, et préférentiellement en C11-C21.

Parmi les acides monocarboxyliques non aromatiques ayant une température de fusion supérieure ou égale à 25°C, susceptibles d'être employés, on peut citer, seul ou en mélange :
- parmi les acides monocarboxyliques saturés : l'acide décanoïque (caprique), l'acide laurique, l'acide tridécanoïque, l'acide myristique, l'acide palmitique, l'acide stéarique, l'acide arachidique, l'acide béhénique, l'acide cérotique (hexacosanoïque);
- parmi les acides monocarboxyliques insaturés mais non aromatiques : l'acide pétrosélinique, l'acide vaccénique, l'acide élaidique, l'acide gondoïque, l'acide gadoléique, l'acide érucique, l'acide nervonique.

De préférence, on peut utiliser l'acide laurique, l'acide palmitique, l'acide stéarique, l'acide béhénique et leurs mélanges, et encore mieux l'acide stéarique ou l'acide béhénique, seuls.

Ledit acide monocarboxylique non aromatique de température de fusion supérieure ou égale à 25°C, ou le mélange desdits acides, représente de préférence 22 à 80% en poids, notamment 25 à 75% en poids, voire 27 à 70% en poids, et mieux 28 à 65% en poids, du poids total du polycondensat final.

Un autre constituant nécessaire pour la préparation des polycondensats selon l'invention est un acide monocarboxylique non aromatique, saturé ou insaturé, linéaire, ramifié et/ou cyclique, comprenant 6 à 32 atomes de carbone, notamment 8 à 28 atomes de carbone et encore mieux 10 à 20, voire 12 à 18, atomes de carbone; et ayant une température de fusion strictement inférieure à 25°C, notamment inférieure à 20°C, voire à 15°C. On peut bien évidemment utiliser un mélange de tels acides monocarboxyliques non aromatiques.

Par acide monocarboxylique non aromatique, on entend un composé de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone.

De préférence, le radical R est saturé. Encore mieux, ledit radical R est linéaire ou ramifié, et préférentiellement en C5-C31.

Parmi les acides monocarboxyliques non aromatiques ayant une température de fusion inférieure à 25°C, susceptibles d'être employés, on peut citer, seul ou en mélange :
- parmi les acides monocarboxyliques saturés : l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide isononanoïque, l'acide isostéarique;
- parmi les acides monocarboxyliques insaturés mais non aromatiques : l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide linoléique, l'acide linolénique, l'acide arachidonique.

De préférence, on peut utiliser l'acide isooctanoïque, l'acide isononanoïque, l'acide isostéarique, et leurs mélanges, et encore mieux l'acide isostéarique seul.

Ledit acide monocarboxylique non aromatique de température de fusion inférieure à 25°C, ou le mélange desdits acides, représente de préférence 0,1 à 35% en poids, notamment 0,5 à 32% en poids, voire 1 à 30% en poids, et mieux 2 à 28% en poids, du poids total du polycondensat final.

De préférence, la quantité totale d'acides monocarboxyliques non aromatiques, à avoir ceux de température de fusion supérieure à 25°C + ceux de température de fusion inférieure à 25°C, est avantageusement comprise entre 30 et 80% en poids, notamment entre 40 et 70% en poids, voire entre 45 et 65% en poids, et mieux entre 50 et 60 % en poids, du poids total du polycondensat final.

Un autre constituant nécessaire pour la préparation des polycondensats selon l'invention est un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone, notamment 2 à 12, voire 3 à 8 atomes de carbone.

On peut bien évidemment utiliser un mélange de tels acides monocarboxyliques aromatiques.

Par acide monocarboxylique aromatique, on entend un composé de formule R'COOH, dans laquelle R' est un radical hydrocarboné aromatique, comprenant 6 à 10 atomes de carbone, et en particulier les radicaux benzoïque et naphtoïque.

Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle.

Parmi les acides monocarboxyliques aromatiques susceptibles d'être employés, on peut citer, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque.

De préférence, on peut utiliser l'acide benzoïque, l'acide 4-tert-butyl-benzoïque, l'acide o-toluique, l'acide m-toluique, l'acide 1-naphtoïque, seuls ou en mélanges; et encore mieux l'acide benzoïque seul.

Ledit acide monocarboxylique aromatique, ou le mélange desdits acides, représente de préférence 0,1 à 10% en poids, notamment 0,5 à 9,95% en poids, mieux encore de 1 à 9,5% en poids, voire 1,5 à 8% en poids, du poids total du polycondensat final.

Un autre constituant nécessaire pour la préparation des polycondensats selon l'invention est un acide polycarboxylique, et/ou un anhydride cyclique d'un tel acide polycarboxylique, et/ou une lactone portant au moins un groupement COOH; ainsi que leurs mélanges.

Ledit acide polycarboxylique peut notamment être choisi parmi les acides polycarboxyliques linéaires, ramifiés et/ou cycliques, saturés ou insaturés, voire aromatiques, comprenant 2 à 50, notamment 2 à 40, atomes de carbone, en particulier 3 à 36, voire 3 à 18, et encore mieux 4 à 12 atomes de carbone, voire 5 à 10 atomes de carbone; ledit acide comprenant au moins deux groupes carboxyliques COOH, de préférence de 2 à 4 groupes COOH; et pouvant comprendre 1 à 10 hétéroatomes, de préférence 1 à 6, identiques ou différents, choisis parmi O, N et S; et/ou pouvant comprendre au moins un radical perfluoré choisi parmi -CF₂- (divalent) ou -CF₃.

De préférence, ledit acide polycarboxylique est aliphatique saturé, linéaire et comprend 2 à 36 atomes de carbone, notamment 3 à 18 atomes de carbone, voire 4 à 12 atomes de carbone; ou bien est aromatique et comprend 8 à 12 atomes de carbone. Il comprend de préférence 2 à 4 groupes COOH.

Ledit anhydride cyclique d'un tel acide polycarboxylique peut notamment répondre à l'une des formules suivantes : 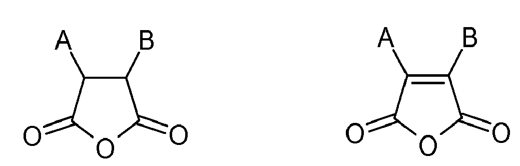 dans lesquelles les groupements A et B sont, indépendamment l'un de l'autre, :
- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 14, notamment 5 à 10, voire 6 à 7 atomes de carbone, saturé ou insaturé, voire aromatique.

De préférence, A et B représentent un atome d'hydrogène ou forment ensemble un cycle aromatique comprenant au total 6 à 10 atomes de carbone.

Parmi les acides polycarboxyliques ou leurs anhydrides, susceptibles d'être employés, on peut citer, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide tétrahydrophtalique, l'acide hexahydrophtalique, l'acide pimélique, l'acide sébacique, l'acide azélaïque, l'acide glutarique, l'acide adipique, l'acide fumarique, l'acide maléïque, l'acide itaconique, les dimères d'acides gras (notamment en C36) tels que les produits commercialisés sous les dénominations Pripol 1006, 1009, 1013 et 1017, par Uniqema;
- les acides tricarboxyliques tels que l'acide cyclohexanetricarboxylique, l'acide trimellitique, l'acide 1,2,3-benzènetricarboxylique, l'acide 1,3,5-benzènetricarboxylique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

De préférence, on peut utiliser l'acide adipique, l'anhydride phtalique et/ou l'acide isophtalique, et encore mieux l'acide isophtalique seul.

On peut également citer les acides polycarboxyliques choisis parmi, seul ou en mélange :
- (i) les acides polycarboxyliques à chaîne saturée ou insaturée, linéaire ou ramifiée, comprenant au moins un hétéroatome choisi parmi O, N et/ou S, notamment 1 à 10 hétéroatomes identiques ou différents, et/ou comprenant au moins un radical perfluoré -CF₂- ou -CF₃ et possédant par ailleurs au moins 2 groupes carboxyliques COOH, notamment 2 à 4 groupes COOH; et/ou un anhydride cyclique d'un tel acide polycarboxylique;
   et/ou
- (ii) les acides polycarboxyliques hétérocycliques, saturé ou insaturé, voire aromatique, comprenant au moins un hétéroatome choisi parmi O, N et/ou S, notamment 1 à 10, voire 1 à 4, hétéroatomes identiques ou différents, et au moins 2 groupes carboxyliques COOH, notamment 2 à 4 groupes COOH; et/ou un anhydride cyclique d'un tel acide polycarboxylique;
   et/ou
- (iii) les acides polycarboxyliques dérivés de sucre, susceptibles d'être obtenus notamment par oxydation d'un aldose, et comprenant au moins 2 groupes carboxyliques COOH, notamment 2 ou 3 groupes COOH; et/ou un anhydride cyclique d'un tel acide polycarboxylique;
   et/ou
- (iv) l'anhydride itaconique et le 1,4-monoanhydride de l'acide 1,4,5,8-naphthalenetetracarboxylique;
   et/ou
- (v) les aminoacides polycarboxyliques (y compris hétérocycliques), c'est-à-dire les acides polycarboxyliques à chaîne saturée ou insaturée, linéaire, ramifiée, et/ou cyclique, comprenant éventuellement au moins un hétéroatome choisi parmi O, N et/ou S, notamment 1 à 10 hétéroatomes identiques ou différents, et/ou comprenant éventuellement au moins un radical perfluoré -CF₂- ou -CF₃, et comprenant en outre au moins une fonction amine primaire, secondaire ou tertiaire (notamment NR1 R2 avec R1 et R2, indépendamment l'un de l'autre choisis parmi H et alkyl en C1-C12), notamment 1 à 3 fonctions amines, identiques ou différentes, et possédant par ailleurs au moins 2 groupes carboxyliques COOH, notamment 2 à 4 groupes COOH; et/ou un anhydride cyclique d'un tel acide polycarboxylique.

On peut tout particulièrement citer, seul ou en mélange, les acides dicarboxyliques suivants :
(i)
   - l'acide 2,2'-[1,5-pentanediylbis(thio)]bis-acétique
   - l'acide 6,6'-[(1,2-dioxo-1,2-ethanediyl)diimino]bis-hexanoïque
   - l'acide 2,2'-sulfinylbis-acétique
   - l'acide 4,13-dioxo- 3,5,12,14-Tetraazahexadecanedioïque
   - Le poly(ethylene glycol) disuccinate, notamment de masse 250-600
   - Le poly(ethylene glycol)bis(carboxymethyl)éther, notamment de masse 250-600
   - Le poly[oxy(1,2-dicarboxy-1,2-ethanediyl)], notamment de DP < 10
   - L'acide 8-[(carboxymethyl)amino]-8-oxo-octanoïque
   - L'acide 2,2'-[methylenebis(sulfonyl)]bis-acétique
   - L'acide 4,4'-(1,6-hexanediyldiimino)bis[4-oxo-butanoïque]
   - L'acide 4,9-dioxo- 3,5,8,10-tétraazadodecanedioïque
   - L'acide 4-[(1-carboxyethyl)amino]-4-oxo-butanoïque
   - L'acide 6-[(3-carboxy-1-oxopropyl)amino]-hexanoïque
   - La N,N'-(1,6-dioxo-1,6-hexanediyl)bis-glycine
   - La N,N'-(1,6-dioxo-1,6-hexanediyl)bis-phenylalanine
   - La N,N'-(1,3-dioxo-1,3-propanediyl)bis-glycine
   - L'acide 4,4'-[(1,4-dioxo-1,4-butanediyl)diimino]bis-butanoïque
   - L'acide 4,4'-[(1,6-dioxo-1,6-hexanediyl)diimino]bis-butanoïque
   - L'acide 6,6'-[1,6-hexanediylbis(iminocarbonylimino)]bis-hexanoïque
   - La N-benzoyl-S-(carboxymethyl)- Cysteine
   - La N,N'-(2,2,3,3-tetrafluoro-1,4-dioxo-1,4-butanediyl)bis- Glycine
   - La N,N'-(2,2,3,3-tetrafluoro-1,4-dioxo-1,4-butanediyl)bis-Alanine
   - L'acide 4,4'-[(2,2,3,3-tetrafluoro-1,4-dioxo-1,4- butanoïque
   - La N,N'-(1,5-dioxo-1,5-pentanediyl)bis- Glycine
   - La N,N'-(1,9-dioxo-1,9-nonanediyl)bis- Glycine
   - La N,N'-(1,10-dioxo-1,10-decanediyl)bis[N-methyl- Glycine
   - Le bis(3-carboxypropyl) ester de l'acide propanedioïque
   - L'acide 7,16-dioxo- 6,8,15,17-Tetraazadocosanedioïque
   - La N-benzoyl-N-(2-carboxyethyl)-Glycine
   - L'acide [2-[(2-carboxymethyl)amino]-2-oxoethyl]- Benzenepropanoïque
   - L'acide [2-[(2-carboxyethyl)amino]-2-oxoethyl]- Benzenepropanoïque
(ii)
   - L'acide 4,7,9,12-Tetraoxapentadecanedioïque
   - L'acide 2,3-Pyridinedicarboxylique
   - L'acide 4-Pyranone-2,6-dicarboxylique
   - L'acide 2,5-Pyrazinedicarboxylique
   - L'acide 2,5-Pyridinedicarboxylique
   - L'acide 2,3-Benzofurandicarboxylique
   - L'acide 7-oxabicyclo[2.2.1]heptane-2,3-dicarboxylique
   - L'acide 3,4-Pyridinedicarboxylique
   - L'acide 2,4-Pyridinedicarboxylique
   - L'acide 3,5-Pyridinedicarboxylique
   - L'acide 2,6-Pyridinedicarboxylique
   - L'acide 1 H-Imidazole-4,5-dicarboxylique
   - L'acide 2,3-Quinolinedicarboxylique
   - L'acide 6,6,7,7-tetrafluoro-3-Oxabicyclo[3.2.0]heptane-2,4-dicarboxylique
   - L'acide 2,6-Pyrazinedicarboxylique
   - L'acide 2,6-Dimethyl-3,5-pyridinedicarboxylique
   - L'acide 1-phenyl-1 H-Pyrazole-3,4-dicarboxylique
   - L'acide 2,5-Furanedicarboxylique
   - L'acide 3,4-Furanedicarboxylique
   - L'acide 1,2,5-Thiadiazole-3,4-dicarboxylique
   - L'acide 1,4-Dihydro-1,2,4,5-tetrazine-3,6-dicarboxylique
   - L'acide 2,3-Furanedicarboxylique
   - L'acide 3,4-Thiophenedicarboxylique
   - L'acide 1H-1,2,3-Triazole-4,5-dicarboxylique
   - L'acide 2-Methylimidazole-4,5-dicarboxylique
   - L'acide 2,4-Quinolinedicarboxylique
   - L'acide Naphtho[2,1-b]furane-1,2-dicarboxylique
   - L'acide 3,4-Quinolinedicarboxylique
   - L'acide 7-Oxabicyclo[2.2.1]hept-5-ene-2,3-dicarboxylique
   - L'acide 2,3-Quinoxalinedicarboxylique
   - L'acide 1,4-Piperazinedicarboxylique
   - L'acide 2,5-dimethyl- 3,4-Furandicarboxylique
   - L'acide tetrahydro-2,5-Thiophenedicarboxylique
   - L'acide 4-phenyl- 3,5-Pyridinedicarboxylique
   - L'acide Thieno[3,2-b]thiophene-2,5-dicarboxylique
   - L'acide 3-methyl- 2,4-Thiophenedicarboxylique
   - L'acide Naphthostyril-5,6-dicarboxylique
   - L'acide 3-phenyl- 2,4-Quinolinedicarboxylique
   - Le 3,4-Dimethyl-2,5-dicarboxythiophene
   - L'acide 3,4-Diphenyl-2,5-thiophenedicarboxylique
   - L'acide 2,5-diphenyl- 3,4-Furanedicarboxylique
   - L'acide 7-oxo-7H-Benzimidazo[2,1-a]benz[de]isoquinoline-3,4-dicarboxylique
   - L'acide 2,3-dihydro-1,3-dioxo-1H-Benz[de]isoquinoline-6,7-dicarboxylique
   - L'acide 3,4-bis(phenylmethoxy)-2,5-Furandicarboxylique
   - L'acide 4,4'-Bibenzoïque-2,2'-sulfone
   - L'acide 2,7-Diphenyl-m-anthrazoline-4,5-dicarboxylique
   - L'acide 2,4-Pyrimidinedicarboxylique
   - L'acide 2-phenyl- 4,5-Thiazoledicarboxylique
   - L'acide 6-phenyl- 2,3-Pyridinedicarboxylique
   - L'acide 5,6-Dimethyl-2,3-pyrazinedicarboxylique
   - L'acide 3,7-Dibenzothiophenedicarboxylique
   - L'acide 9-oxo-9H-Xanthene-1,7-dicarboxylique
   - L'acide 2-(1,1-dimethylethyl)-H-Imidazole-4,5-dicarboxylique
   - L'acide 6,7-Quinolinedicarboxylique
   - L'acide 6-Methyl-2,3-pyridinedicarboxylique
   - L'acide 4,5-Pyrimidinedicarboxylique
   - L'acide 2-methyl-3,4-Furanedicarboxylique
   - L'acide 1,2-Indolizinedicarboxylique
   - L'acide 2,8-Dibenzothiophenedicarboxylique
   - L'acide 3,6-Pyridazinedicarboxylique
   - L'acide 1,10-Phenanthroline-2,9-dicarboxylique
   - L'acide 1,4,5,6-tetrahydro-5,6-dioxo-2,3-Pyrazinedicarboxylique
   - L'acide 3,4-Dimethoxy-2,5-furandicarboxylique
   - L'acide 2-Ethyl-4,5-imidazoledicarboxylique
   - L'acide 2-Propyl-1 H-imidazole-4,5-dicarboxylique
   - L'acide 4-phenyl- 2,5-Pyridinedicarboxylique
   - L'acide 4,5-Pyridazinedicarboxylique
   - L'acide 1,4,5,8-tetrahydro-1,4:5,8-Diepoxynaphthalene-4a,8a-dicarboxylique
   - L'acide 5,5-dioxide-2,8-Dibenzothiophenedicarboxylique
   - L'acide Pyrazolo[1,5-a]pyridine-2,3-dicarboxylique
   - L'acide 2,3-dihydro-1H-Pyrrolizine-1,7-dicarboxylique
   - L'acide 6-methyl-2,4,5-Pyridinetricarboxylique
   - L'acide Pyrrolo[2,1,5-cd]indolizine-5,6-dicarboxylique
   - L'acide 3,4-bis(2,2,3,3,4,4,4-heptafluorobutyl)-1H-Pyrrole-2,5-dicarboxylique
   - L'acide 6,7,9,10,17,18,20,21-octahydrodibenzo[b,k][1,4,7,10,13,16]hexaoxacyclooctadecin-2,14-dicarboxylique
   - L'acide 6,7,9,10,17,18,20,21-octahydro-dibenzo[b,k][1,4,7,10,13,16]hexaoxacyclo octadecin-2,13-dicarboxylique
   - L'acide 2-methyl- 3,4-Quinolinedicarboxylique
   - L'acide 4,7-Quinolinedicarboxylique
   - L'acide 3,5-Isoxazoledicarboxylique
   - L'acide 2-(trifluoromethyl)- 3,4-Furanedicarboxylique
   - L'acide 5-(trifluoromethyl)- 2,4-Furanedicarboxylique
   - L'acide 6-methyl-2,4-quinolinedicarboxylique
   - L'acide 5-oxo- 1,2-Pyrrolidinedicarboxylique
   - L'acide 5-Ethyl-2,3-pyridinedicarboxylique
   - L'acide 1,2-dihydro-2-oxo- 3,4-quinolinedicarboxylique
   - L'acide 4,6-Phenoxathiindicarboxylique
   - L'acide 10,10-dioxide 1,9-phenoxathiindicarboxylique
   - L'acide 3,4-dihydro-2H-1,4-Thiazine-3,5-dicarboxylique
   - L'acide 2,7-Di(tert-butyl)-9,9-dimethyl-4,5-xanthenedicarboxylique
   - L'acide 6-methyl-2,3-Quinoxalinedicarboxylique
   - L'acide 3,7-Quinolinedicarboxylique
   - L'acide 2,5-Quinolinedicarboxylique
   - L'acide 2-Methyl-6-phenyl-3,4-pyridinedicarboxylique
   - L'acide 3,4-dimethyl- thieno[2,3-b]thiophene-2,5-dicarboxylique
   - L'acide 3,4-Dimethoxythiophene-2,5-dicarboxylique
   - L'acide 5-methyl-3,4-Isoxazoledicarboxylique
   - L'acide 2,6-bis(aminocarbonyl)-3,5-Pyridinedicarboxylique
   - L'acide 3,5-bis(aminocarbonyl)-2,6-Pyrazinedicarboxylique
   - L'acide 2,3-Pyridinedicarboxylique
   - L'acide 6-(1,1-dimethylethyl)-2-ethyl-3,4-Pyridinedicarboxylique
   - L'acide 3-methyl-5-phenyl-2,4-Thiophenedicarboxylique
   - L'acide 1,2-dihydro-2-oxo-6-phenyl-3,5-Pyridinedicarboxylique
   - L'acide 8-methyl-2,4-Quinolinedicarboxylique
   - L'acide 4-ethyl-2,6-dimethyl-3,5-Pyridinedicarboxylique
   - L'acide 5-(phenoxymethyl)-2,4-Furanedicarboxylique
   - L'acide 5-(acetylamino)-3-methyl-2,4-Thiophenedicarboxylique
   - L'acide 2-(4-heptylphenyl)- 4,8-Quinolinedicarboxylique
   - L'acide 2,8-bis(4-heptylphenyl)-pyrido[3,2-g]quinoline-4,6-dicarboxylique
   - L'acide 1,2,3,4,6,7,8,9-octahydro-2,8-dioxo-pyrido[3,2]quinoline-3,7-dicarboxylique
   - L'acide 2,8-dimethyl- Pyrido[3,2-g]quinoline-3,7-dicarboxylique
   - L'acide 5,6-Quinolinedicarboxylique
   - L'acide 6-ethyl-2-methyl- Cinchomeronique
   - L'acide 2-methyl-6-propyl-Cinchomeronique
   - L'acide 6-isopropyl-2-methyl- Cinchomeronique
   - L'acide 6-tert-butyl-2-methyl- Cinchomeronique
   - L'acide 1,4-dimethyl-7-Oxabicyclo[2.2.1]heptane-2,3-dicarboxylique
   - L'acide 1,2-dihydro-2-oxo- 3,8-Quinolinedicarboxylique
   - L'acide 1,2-dihydro-2-oxo-3,6-Quinolinedicarboxylique
   - L'acide 1,2-dihydro-2-oxo-3,7-Quinolinedicarboxylique
   - L'acide 3,7-dimethyl-2,8-diphenyl-Pyrido[3,2-g]quinoline-4,6-dicarboxylique
   - L'acide 8-methyl-2,3-Quinolinedicarboxylique
   - L'acide 3-[[(1,1-dimethylethyl)amino]sulfonyl]-2,5-Thiophene-dicarboxylique
   - L'acide 4-(acetylamino)-2,3-Thiophenedicarboxylique
   - L'acide 2,5-Pyridinedicarboxylique
   - L'acide 2,6-Pyridinedicarboxylique
   - L'acide 2,4-Thiophenedicarboxylique
   - L'acide 2,5-Thiophenedicarboxylique
   - L'acide 1,4-pyrane-2,6-dicarboxylique
(iii)
   - L'acide Ribarique
   - L'acide Glucarique
   - L'acide Xylarique
   - L'acide Arabinarique
   - L'acide Mannarique
   - L'acide Idarique
   - L'acide Altrarique
   - L'acide L-Glucarique
   - L'acide L-Arabinarique
   - L'acide Allarique
   - L'acide Galactarique
   - L'acide meso-Tartarique
   - L'acide D-Glucarique
   - L'acide L-Idarique
   - L'acide Hexarique
   - L'acide 2,3-dihydroxy- butanedioique
   - L'acide D-Tartarique
   - L'acide DL-Tartarique
   - L'acide D-Glucarique
   - l'acide tartarique
   - L'acide Tetrahydroxysuccinique
   - L'acide 2-carboxy-2,3-dideoxy D-manno-2-Octulopyranosonique
   - L'acide methyl 3-deoxy-D-arabino-2-Heptulopyranosarique
   - L'acide D-lyxo-2-Heptulopyranosarique
   - L'acide 2,6-anhydro-L-glycero-L-galacto-Heptarique
(iv)
   - le 1,4-monoanhydride de l'acide 1,4,5,8-Naphthalenetetracarboxylique
   - l'anhydride itaconique
(v)
   - l'acide 1,4-dihydro-4-oxo-2,6-Pyridinedicarboxylique
   - l'acide 2,6-Piperidinedicarboxylique
   - l'acide 1 H-Pyrrole-3,4-dicarboxylique
   - l'acide 4-amino-2,6-dicarboxylique
   - l'acide 1-methyl-1 H-Pyrazole-3,4-dicarboxylique
   - l'acide 2,3-Piperidinedicarboxylique
   - l'acide 1-methyl-1 H-Imidazole-4,5-dicarboxylique
   - l'acide 2,4-Thiazolidinedicarboxylique
   - l'acide 1-(phenylmethyl)-1 H-Imidazole-4,5-dicarboxylique
   - l'acide 5-amino-6-oxo-2,3-Piperidinedicarboxylique
   - l'acide 5-amino-6-oxo-2,4-Piperidinedicarboxylique
   - l'acide 5-amino-6-oxo-2,3-Piperidinedicarboxylique
   - l'acide 5-amino-6-oxo-, [2S-(2α,4β,5α)]- 2,4-Piperidinedicarboxylique
   - l'acide (2S,4R)-2,4-Pyrrolidinedicarboxylique
   - l'acide-(2S-cis)-2,4-Pyrrolidinedicarboxylique
   - l'acide 2-amino-1 H-Imidazole-4,5-dicarboxylique
   - l'acide 2,5-Pyrrolidinedicarboxylique
   - l'acide 4-amino-3,5-Isothiazoledicarboxylique
   - l'acide 1-methyl- 1 H-Pyrazole-3,5-dicarboxylique
   - l'acide 7-(diethylamino)-2-oxo-2H-1-Benzopyran-3,4-dicarboxylique
   - l'acide 3,4-diethyl-1 H-Pyrrole-2,5-dicarboxylique
   - l'acide 1-phenyl-1 H-Pyrrole-3,4-dicarboxylique
   - l'acide cis-2,3-Piperazinedicarboxylique
   - l'acide 2,3-Piperazinedicarboxylique
   - l'acide 2,5-Piperazinedicarboxylique
   - l'acide 2,6-Piperazinedicarboxylique
   - l'acide 2-amino-3,5-Pyridinedicarboxylique
   - l'acide 2-methyl-Pyrrole-3,4-dicarboxylique
   - l'acide 4-(methylamino)-2,6-Pyridinedicarboxylique
   - l'acide 2-amino-6-methyl-3,4-Pyridinedicarboxylique
   - l'acide 5-amino-2-methyl-3,4-Pyridinedicarboxylique
   - l'acide 2-amino-6-methyl- 3,5-Pyridinedicarboxylique
   - l'acide 2,5-dimethyl-Pyrrole-3,4-dicarboxylique
   - l'acide 2,5-dimethyl-Pyrrole-3,4-dicarboxylique
   - l'acide 2-amino-6-hydroxy- 3,5-Pyridinedicarboxylique
   - l'acide 2,4-Pyrrolidinedicarboxylique
   - l'acide 1 H-Indole-2,4-dicarboxylique
   - l'acide 1 H-Indole-2,6-dicarboxylique
   - l'acide 1 H-Indole-2,5-dicarboxylique
   - l'acide 5-phenyl-2,4-Pyrrolidinedicarboxylique
   - l'acide 5-methyl-2,4-Pyrrolidinedicarboxylique
   - l'acide trans-2,4-Azetidinedicarboxylique
   - l'acide cis-2,4-Azetidinedicarboxylique
   - l'acide 3,5-Piperidinedicarboxylique
   - l'acide 2,3-Pyrrolidinedicarboxylique
   - l'acide 2,3-Azetidinedicarboxylique
   - l'acide 3,4-Pyrrolidinedicarboxylique
   - l'acide 2,3-dihydro-6H-1,4-Dioxino[2,3-c]pyrrole-5,7-dicarboxylique
   - l'acide 1 H-Imidazole-2,4-dicarboxylique
   - l'acide 1-butyl-1 H-Pyrrole-2,3-dicarboxylique
   - l'acide 3-amino-1-oxide-2,4-Pyridinedicarboxylique
   - l'acide 2,3-dihydro-5-phenyl-1 H-Pyrrolizine-6,7-dicarboxylique
   - l'acide 3a,4,5,9b-tetrahydro-3H-Cyclopenta[c]quinoline-4,6-dicarboxylique
   - l'acide 3a,4,5,9b-tetrahydro-3H-Cyclopenta[c]quinoline-4,8-dicarboxylique
   - l'acide 2,3-dihydro-1H-Imidazole-4,5-dicarboxylique
   - l'acide 5-amino-6-methyl-Lutidinique
   - l'acide 1 H-Indole-3,7-dicarboxylique
   - l'acide 3,3-dimethyl-2,6-Piperidinedicarboxylique
   - l'acide 1-butyl-2,5-Pyrrolidinedicarboxylique
   - l'acide 1 H-Indole-4,6-dicarboxylique
   - l'acide 1-(phenylmethyl)-3,4-Pyrrolidinedicarboxylique
   - l'acide 3-(carboxymethyl)-1 H-Indole-2,6-dicarboxylique
   - l'acide 3,4-bis(2,2,2-trifluoroethyl)-1H-Pyrrole-2,5-dicarboxylique
   - l'acide 9-hexyl-9H-Carbazole-3,6-dicarboxylique
   - l'acide 3-methyl-5-(1-piperazinylsulfonyl)-2,4-Thiophenedicarboxylique
   - l'acide 2,3,4,9-tetrahydro-1H-Carbazole-5,7-dicarboxylique
   - l'acide 2,3-dimethyl-1 H-Indole-4,6-dicarboxylique
   - l'acide 7-amino-1,4-dihydro-4-oxo-3,6-Quinolinedicarboxylique
   - l'acide 5-amino-3-methyl-2,4-Thiophenedicarboxylique
   - l'acide (m-tolylimino)di-Acetique
   - l'acide (o-tolylimino)di-Acetique
   - la D-Cystathionine
   - l'acide Phenethyliminodiacetique
   - l'acide 2-Benzyl-2,2'-iminodiacetique
   - la L-α-glutamyl-L-alanyl-L-Alanine
   - l'acide N,N'-Dibenzylethylenediaminediacetique
   - la N-L-y-glutamyl-D-Alanine
   - la Glycyl-L-glutamyl glycine
   - la N-(carboxymethyl)-N-(tetrahydro-1,1-dioxido-3-thienyl)-Glycine
   - la N-(2-carboxyethyl)-N-phenyl-beta-Alanine
   - la N-(carboxymethyl)-N-octyl-glycine
   - l'acide N-(tert-Butoxycarbonyl)iminodiacetique
   - la N-(carboxymethyl)-L-Alanine
   - la N-(6-aminohexyl)-N-(carboxymethyl)-Glycine
   - la N-(carboxymethyl)-N-tetradecyl-Glycine
   - la N-(1-carboxyethyl)-D-Alanine
   - la N-(carboxymethyl)-D-Alanine
   - l'acide decyliminodiacetique
   - l'acide 3,3'-(dimethylhydrazono)bis-propanoique
   - la N-(carboxymethyl)-N-[2-(2,6-dioxo-4-morpholinyl)ethyl]-glycine
   - la N-alpha-aspartyl-glycine
   - la N-beta-aspartyl-glycine
   - la N-L-alpha-aspartyl-beta-Alanine
   - l'acide 3,4-Xylylamino-N,N-diacetique
   - la N-(1-carboxyethyl)-Alanine
   - la N-(carboxymethyl)-Alanine
   - la N,N'-methylenebis-glycine
   - la N-(aminomethyl)-N-(carboxymethyl)-glycine
   - la N-(aminomethyl)-N-(carboxymethyl)-glycine
   - l'acide 2,2'-(methylhydrazono)bis-acetique
   - la N-(2-carboxyethyl)-N-(4-methylphenyl)-beta-Alanine
   - la N-(2-carboxyethyl)-N-(3-methylphenyl)-beta-Alanine
   - la 3-[(carboxymethyl)amino]-Alanine
   - la D-alpha-aspartyl-D-Alanine
   - la N-(2-carboxyethyl)-N-(1-oxohexadecyl)-beta-Alanine
   - la N-(2-carboxyethyl)-N-(1-oxodecyl)-beta-Alanine
   - la N-(2-carboxyethyl)-N-(1-oxotetradecyl)-beta-Alanine
   - l'acide amino[(carboxymethyl)thio]- Acetique
   - la N,N'-1,6-hexanediylbis-beta-Alanine
   - la N-(carboxymethyl)-N-phenyl-beta-Alanine
   - la N-(1-carboxyethyl)- L-Alanine
   - l'acide L-glutamique
   - l'acide L-aspartique.

On peut également citer, seul ou en mélange, les acides tricarboxyliques et tétracarboxyliques suivants, ainsi que leurs anhydrides:
- L'acide 3,3',3"-[1,2,3-propanetriyltris(oxy)]tris-propanoïque
- L'acide Pyrazinetricarboxylique
- L'acide 4-(3-carboxyphenyl)-2,5-Pyridinedicarboxylique
- L'acide 3-(carboxymethyl)-2,4-Quinolinedicarboxylique
- L'acide 3-(carboxymethyl)-1 H-Indole-2,5-dicarboxylique
- L'acide 3-C-carboxy-2-deoxy-D-threo-Pentarique
- L'acide Hydroxycitrique
- L'acide D-glucopyranuronosyl-D-arabino-2-Hexulofuranosidarique
- L'acide 2,3,5,6-Pyridinetetracarboxylique
- la N,N'-1,2-ethanediylbis[N-(carboxymethyl)- β-Alanine
- l'acide L-α-aspartyl-L-Aspartique
- l'acide 4-[bis(carboxymethyl)amino]-Benzoique
- l'acide 7-[bis(carboxymethyl)amino]-Heptanoique
- l'acide N-(2-carboxyethyl)-Aspartique
- l'acide 3-[bis(2-carboxyethyl)amino]-Benzoique
- l'acide 4-[bis(2-carboxyethyl)amino]-Benzoique.

De préférence, on peut utiliser l'acide 6,6'-[(1,2-dioxo-1,2-ethanediyl)diimino]bis-hexanoïque, l'acide 2,2'-sulfinylbis-acétique, l'acide 4,13-dioxo-3,5,12,14-tetraazahexadecanedioïque, le poly(ethylene glycol)disuccinate, le poly(ethylene glycol)bis(carboxymethyl)éther, l'acide 8-[(carboxymethyl)amino]-8-oxo-octanoïque, l'acide 2,2'-[methylenebis(sulfonyl)]bis-acétique, l'acide 4,4'-(1,6-hexanediyldiimino)bis[4-oxo-butanoïque], l'acide 4,9-dioxo-3,5,8,10-tétraazadodecanedioïque, l'acide 4-[(1-carboxyethyl)amino]-4-oxo-butanoïque, l'acide 6-[(3-carboxy-1-oxopropyl)amino]-hexanoïque, la N,N'-(1,6-dioxo-1,6-hexanediyl)bis-glycine, la N,N'-(1,3-dioxo-1,3-propanediyl)bis-glycine, l'acide 4,7,9,12-tétraoxapentadecanedioïque, l'acide 4-Pyranone-2,6-dicarboxylique, l'acide 2,5-Pyrazinedicarboxylique, l'acide 1H-Imidazole-4,5-dicarboxylique, l'acide 2,6-Pyrazinedicarboxylique, l'acide 2,5-Furanedicarboxylique, l'acide 3,4-Furanedicarboxylique, l'acide 2,3-Furanedicarboxylique, l'acide 2,5-diphenyl-3,4-Furanedicarboxylique, l'acide 2-methyl-3,4-Furanedicarboxylique, l'acide D-Tartarique, l'acide DL-Tartarique, l'acide L-Tartarique, l'acide Galactarique, l'acide D-Glucarique; L'acide 2,5-Pyridinedicarboxylique, l'acide 2,5-Pyrrolidinedicarboxylique, l'acide 1-phenyl-1H-Pyrrole-3,4-dicarboxylique, l'acide 2,4-Pyrrolidinedicarboxylique, l'acide 5-phenyl-2,4-Pyrrolidinedicarboxylique, l'acide 3,5-Piperidinedicarboxylique, l'acide 3,4-Pyrrolidinedicarboxylique, l'acide 1-butyl-2,5-Pyrrolidinedicarboxylique, l'acide 1-(phenylmethyl)-3,4-Pyrrolidinedicarboxylique, la N-(2-carboxyethyl)-N-phenyl-β-Alanine, la N-(carboxymethyl)-N-octyl-glycine, la N-(1-carboxyethyl)-L-Alanine, l'acide L-glutamique, l'acide L-aspartique, l'acide N-(2-carboxyethyl)-Aspartique; et leurs mélanges.

On préférera tout particulièrement l'acide 2,2'-sulfinylbis-acétique, l'acide 2,2'-[methylenebis(sulfonyl)]bis-acétique, la N,N'-(1,3-dioxo-1,3-propanediyl)bis-glycine, l'acide 2,5-Furanedicarboxylique, l'acide D-Tartarique, l'acide DL-Tartarique, l'acide L-Tartarique, l'acide Galactarique, l'acide L-glutamique, l'acide L-aspartique, et leurs mélanges.

On peut également utiliser une lactone comprenant au moins un groupement carboxylique, notamment 1, 2 ou 3 groupements COOH. De préférence, les lactones comprennent 5 à 14 atomes de carbone, notamment 6 à 13, voire 6 à 12 atomes de carbone.

On peut tout particulièrement citer, seul ou en mélange, les lactones suivantes :
- L'acide tetrahydro-2,2-dimethyl-5-oxo-3-Furancarboxylique
- L'acide 4,7,7-trimethyl-3-oxo-2-Oxabicyclo[2.2.1]heptane-1-carboxylique
- L'acide 4,6-dimethyl-2-oxo-2H-Pyran-5-carboxylique
- L'acide 2-oxo-2H-Pyran-5-carboxylique-2-Pentenedioique
- L'acide 2-oxo-2H-1-Benzopyran-3-carboxylique
- l'acide 2-oxo-2H-Pyran-6-carboxylique
- L'acide 1,3-dihydro-3-oxo-1-Isobenzofurancarboxylique
- L'acide 4-methyl-2-oxo-2H-1-Benzopyran-3-carboxylique
- L'acide 1-oxo-1H-2-Benzopyran-3-carboxylique
- L'acide 8-methoxy-2-oxo-2H-1-Benzopyran-3-carboxylique
- L'acide 2-oxo-1-Oxaspiro[4.5]decane-4-carboxylique
- L'acide 2-oxo-2H-Pyran-3-carboxylicique
- L'acide 4-methyl-2-oxo-2H-Pyran-6-carboxylique
- L'acide 3-oxo-3H-Naphtho[2,1-b]pyran-2-carboxylique
- L'acide tetrahydro-5-oxo-2,3-furandicarboxylique
- L'acide 1,3-dihydro-3-oxo-4-Isobenzofurancarboxylique
- L'acide 1,3-dihydro-1-oxo- 5-Isobenzofurancarboxylique
- L'acide hexahydro-2-oxo-3,5-Methano-2H-cyclopenta[b]furan-7-carboxylique
- L'acide 6-methyl-2,4-dioxo-2H-Pyran-5-carboxylique
- L'acide 1-oxo-3-Isochromancarboxylique
- L'acide 2-oxo-2H-1-Benzopyran-6-carboxylique
- L'acide 6-methyl-2-oxo-2H-1-Benzopyran-3-carboxylique
- L'acide 2,5-dihydro-4,5,5-trimethyl-2-oxo-3-Furancarboxylique
- L'acide tetrahydro-5-oxo-2-phenyl- 3-Furancarboxylique
- L'acide tetrahydro-5-oxo-4-propyl-2-Furoïque
- L'acide 2-butyl-2,3-dideoxy-pentarique
- L'acide 2-oxo 2H-1-Benzopyran-7-carboxylique
- L'acide 2-oxo-1-Oxaspiro[4.4]nonane-4-carboxylique
- L'acide 4-ethyltetrahydro-5-oxo-2-furoïque
- L'acide 5-ethyltetrahydro-2,3-dimethyl-6-oxo-2H-Pyran-2-carboxylique
- L'acide 7-methoxy-2-oxo-2H-1-benzopyran-3-carboxylique
- L'acide 2-oxo-2H-1-Benzopyran-4-carboxylique
- L'acide 2-oxo-6-pentyl-2H-Pyran-3-carboxylique
- L'acide 7-oxo-4-Oxepanecarboxylique
- L'acide 3-(carboxymethyl)-2,3-dideoxy-pentarique
- L'acide 2,3-dihydro-2-oxo- 7-benzofurancarboxylique
- L'acide 1,3,4,5-tetrahydro-1-oxo-2-benzoxepin-7-carboxylique
- L'acide 3,4-dihydro-3-oxo-1 H-2-benzopyran-6-carboxylique
- L'acide 2,3,4,5-tetrahydro-2-oxo-1-Benzoxepin-7-carboxylique
- L'acide 3,4-dihydro-1-oxo-1H-2-benzopyran-8-carboxylique
- L'acide 1,3,4,5-tetrahydro-3-oxo-2-benzoxepin-9-carboxylique
- L'acide 1,3,4,5-tetrahydro-3-oxo-2-Benzoxepin-7-carboxylique
- L'acide 3,4-dihydro-2-oxo- 2H-1-benzopyran-8-carboxylique
- L'acide 1,3,4,5-tetrahydro-1-oxo-2-benzoxepin-9-carboxylicique
- L'acide 3,4-dihydro-1-oxo-1H-2-benzopyran-6-carboxylique
- L'acide 3,4-dihydro-3-oxo-1 H-2-benzopyran-8-carboxylique
- L'acide 2,3,4,5-tetrahydro-2-oxo-1-benzoxepin-9-carboxylique
- La lactone de l'acide isocitrique
- L'acide 5-oxo-2-tetrahydrofuranecarboxylique.

De préférence, on peut utiliser l'acide tetrahydro-5-oxo-2,3-furandicarboxylique, l'acide 1,3-dihydro-3-oxo-4-Isobenzofurancarboxylique, l'acide 1,3-dihydro-1-oxo- 5-Isobenzofurancarboxylique, l'acide tetrahydro-5-oxo-2-phenyl-3-furanecarboxylique, la lactone de l'acide isocitrique, l'acide 5-oxo-2-tetrahydrofuranecarboxylique et leurs mélanges.

Ledit acide polycarboxylique et/ou son anhydride cyclique et/ou la lactone, et leurs mélanges, représente de préférence 1 à 40% en poids, voire 5 à 35% en poids, notamment 10 à 30% en poids, et mieux 14 à 25% en poids, du poids total du polycondensat final.

Dans un mode de réalisation préféré de l'invention, l'acide monocarboxylique aromatique est présent en quantité molaire inférieure ou égale à celle des acides monocarboxyliques non aromatiques (de haut et bas points de fusion); notamment le rapport entre le nombre de mole d'acide monocarboxylique aromatique et le nombre de mole total d'acides monocarboxyliques non aromatiques est de préférence compris entre 0,08 et 0,70, notamment entre 0,10 et 0,60, en particulier entre 0,12 et 0,40.

On a constaté que cela permet notamment d'obtenir un polymère avantageusement soluble dans les milieux huileux généralement employés pour formuler des compositions cosmétiques de type rouges à lèvres ou fonds de teint; par ailleurs, le film obtenu présente une rigidité et une souplesse adéquates pour son utilisation dans ce type de formulation, tout en ayant une brillance et une tenue de la brillance telles que recherchées.

De préférence, le polycondensat selon l'invention est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
   présent de préférence en une quantité de 10 à 30% en poids, notamment 12 à 25% en poids, et mieux 14 à 22% en poids, par rapport au poids total du polycondensat final;
- d'au moins un acide monocarboxylique non aromatique ayant une température de fusion supérieure ou égale à 25°C, choisi parmi, seul ou en mélange, l'acide décanoïque, l'acide laurique, l'acide tridécanoïque, l'acide myristique, l'acide palmitique, l'acide stéarique, l'acide arachidique, l'acide béhénique, l'acide cérotique, l'acide pétrosélinique, l'acide vaccénique, l'acide élaidique, l'acide gondoïque, l'acide gadoléique, l'acide érucique, l'acide nervonique;
   présent de préférence en une quantité de 22 à 80% en poids, notamment 25 à 75% en poids, et mieux 27 à 70% en poids, par rapport au poids total du polycondensat final;
- d'au moins un acide monocarboxylique non aromatique ayant une température de fusion inférieure à 25°C, choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide isononanoïque, l'acide isostéarique; l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide linoléique, l'acide linolénique, l'acide arachidonique.
   présent de préférence en une quantité de 0,1 à 35% en poids, notamment 0,5 à 32% en poids, et mieux 1 à 30% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
   présent de préférence en une quantité de 0,1 à 10% en poids, notamment 1 à 9,5% en poids, voire 1,5 à 8% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque, l'anhydride succinique, l'acide 2,2'-sulfinylbis-acétique, l'acide 2,2'-[methylenebis(sulfonyl)]bis-acétique, la N,N'-(1,3-dioxo-1,3-propanediyl)bis-glycine, l'acide 2,5-Furanedicarboxylique, l'acide D-Tartarique, l'acide DL-Tartarique, l'acide L-Tartarique, l'acide Galactarique et leurs mélanges;
   présent de préférence en une quantité de 1 à 40% en poids, notamment 10 à 30% en poids, et mieux 14 à 25% en poids, par rapport au poids total du polycondensat final.

Préférentiellement, le polycondensat selon l'invention est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 10 à 30% en poids, notamment 12 à 25% en poids, et mieux 14 à 22% en poids, par rapport au poids total du polycondensat final;
- d'au moins un acide monocarboxylique non aromatique choisi parmi, seul ou en mélange, l'acide laurique, l'acide palmitique, l'acide stéarique, l'acide béhénique et leurs mélanges, et encore mieux l'acide stéarique seul;
   présent de préférence en une quantité de 22 à 80% en poids, notamment 25 à 75% en poids, et mieux 27 à 70% en poids, par rapport au poids total du polycondensat final;
- d'au moins un acide monocarboxylique non aromatique choisi parmi, seul ou en mélange, l'acide isooctanoïque, l'acide isononanoïque, l'acide isostéarique; présent de préférence en une quantité de 0,1 à 35% en poids, notamment 0,5 à 32% en poids, et mieux 1 à 30% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 0,1 à 10% en poids, notamment 1 à 9,5% en poids, voire 1,5 à 8% en poids par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 1 à 40% en poids, notamment 10 à 30% en poids, et mieux 14 à 25% en poids, par rapport au poids total du polycondensat final.

Dans un mode de réalisation préféré de l'invention, le polycondensat est susceptible d'être préparé exclusivement (ou uniquement) à partir des monomères/catégories de monomères mentionnés dans la présente description, c'est-à-dire qu'il ne comprend pas d'autres monomères/catégories de monomères additionnels; dans ce cas, ledit polycondensat est donc constitué exclusivement (ou uniquement) de 10-30% en poids de polyol comprenant 3 à 6 groupes hydroxyles; de 22-80% en poids d'acide monocarboxylique non aromatique, saturé ou insaturé, linéaire, ramifié et/ou cyclique, comprenant 10-32 atomes de carbone, et ayant une température de fusion supérieure ou égale à 25°C; de 0,1-35% en poids, d'acide monocarboxylique non aromatique, saturé ou insaturé, linéaire, ramifié et/ou cyclique, comprenant 6 à 32 atomes de carbone, et ayant une température de fusion strictement inférieure à 25°C; de 0,1-10% en poids d'acide monocarboxylique aromatique comprenant 7-11 atomes de carbone, éventuellement substitué par 1-3 radicaux alkyles, saturés ou insaturés, linéaires, ramifiés et/ou cycliques, qui comprennent 1-32 atomes de carbone; de 1-40% en poids d'acide polycarboxylique, saturé ou insaturé, voire aromatique, linéaire, ramifié et/ou cyclique, comprenant au moins 2 groupes carboxyliques COOH, pouvant comprendre des hétéroatomes; et/ou un anhydride cyclique d'un tel acide polycarboxylique; et/ou une lactone comprenant au moins un groupement COOH.

De préférence, le polycondensat selon l'invention présente :
- un indice d'acide, exprimé en mg d'hydroxyde de potassium par g de polycondensat, supérieur ou égal à 1; notamment compris entre 2 et 30, et encore mieux compris entre 2,5 et 15; et/ou
- un indice d'hydroxyle exprimé en mg d'hydroxyde de potassium par g de polycondensat, supérieur ou égal à 30; notamment compris entre 30 et 120, et encore mieux compris entre 40 et 90.

Ces indices d'acide et d'hydroxyle peuvent être aisément déterminés par l'homme du métier par les méthodes analytiques habituelles.

De préférence, le polycondensat selon l'invention présente une viscosité, mesurée à 110°C, comprise entre 20 et 4000 mPa.s, notamment entre 30 et 3500 mPa.s, voire entre 40 et 3000 mPa.s et encore mieux entre 50 et 2500 mPa.s. Cette viscosité est mesurée de la manière décrite avant les exemples.

De préférence, le polycondensat selon l'invention présente une masse moléculaire moyenne en poids (Mw) comprise entre 1500 et 300 000, voire entre 2000 et 200 000, et notamment entre 3000 et 100 000.

Le poids moléculaire moyen peut être déterminé par chromatographie par perméation sur gel ou par diffusion de la lumière, selon la solubilité du polymère considéré.

Par ailleurs, le polycondensat est avantageusement soluble dans les milieux huileux cosmétiques usuellement employés, et notamment dans les huiles végétales, les alcanes, les esters gras, les alcools gras, les huiles de silicone, et plus particulièrement dans les milieux comprenant l'isododécane, le Parléam, l'isononanoate d'isononyle, l'octyldodécanol, la phényl triméthicone et/ou le benzoate d'alkyle en C12-C15.

Par soluble, on entend que le polymère forme une solution limpide dans au moins un solvant choisi parmi l'isododécane, le Parléam, l'isononanoate d'isononyle, l'octyldodécanol et le benzoate d'alkyle en C12-C15, à raison d'au moins 50% en poids, à 70°C. Certains composés présentent même une solubilité particulièrement avantageuse dans certains domaines d'applications, à savoir une solubilité dans au moins un des solvants cités ci-dessus, à raison d'au moins 50% en poids, à 25°C.

Le polycondensat selon l'invention peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et/ou l'anhydride cyclique et/ou la lactone, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm).

Il est également possible de réaliser la réaction, en tout ou en partie, dans un solvant inerte tel que le xylène et/ou sous une pression réduite, pour faciliter l'élimination de l'eau.

Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1 %, par rapport au poids total de monomères, de façon à limiter les éventuelles dégradations liées à un chauffage prolongé.

L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.

Parmi les antioxydants particulièrement préférés, on peut notamment citer le BHT, le BHA , le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxy-phenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N,N-hexamethylene bis(3,5-di-tertbutyl-4-hydroxyhydrocinnamamide), le pentaerythritol tetrakis (3-(3,5-di-tertbutyl-4-hydroxyphenyl)propionate) notamment celui commercialisé par CIBA sous le nom IRGANOX 1010; l'octadecyl 3-(3,5-di-tertbutyl-4-hydroxphenyl) propionate notamment celui commercialisé par CIBA sous le nom IRGANOX 1076; la 1,3,5-tris(3,5-di-tertbutyl-4-hydroxybenzyl)-1,3,5-triazine-2,4,6(1H,3H,5H)trione notamment celui commercialisée par Mayzo of Norcross, Ga sous le nom BNX 3114; le di(stearyl)pentaerythritol diphosphite, le tris(2,4-ditertbutyl phenyl)phosphite notamment celui commercialisé par CIBA sous le nom IRGAFOS 168; le dilauryl thiodipropionate notamment celui commercialisé par CIBA sous le nom IRGANOX PS800; le bis(2,4-di-tertbutyl)pentaerythritol diphosphite notamment celui commercialisé par CIBA sous le nom IRGAFOS 126; le bis(2,4-bis)[2-phénylpropan-2-yl]phényl)pentaérythritol diphosphite, le triphénylphosphite, le (2,4-di-tertbutylphenyl)pentaerythritol diphosphite notamment celui commercialisé par GE Specialty Chemicals sous le nom ULTRANOX 626; le tris(nonylphenyl)phosphite notamment celui commercialisé par CIBA sous le nom IRGAFOS TNPP; le mélange 1:1 de N,N-hexamethylenebis(3,5-di-tertbutyl-4-hydroxy-hydrocinnamamide) et de tris(2,4-di-tertbutylphenyl)phosphate notamment celui commercialisé par CIBA sous le nom Irganox B 1171; le tétrakis (2,4-di-tert-butylphényl)phosphite notamment celui commercialisé par CIBA sous le nom IRGAFOS P-EPQ; le distéarylthiodipropionate notamment celui commercialisé par CIBA sous le nom IRGANOX PS802; le 2,4-bis(octylthiométhyl)o-crésol notamment celui commercialisé par CIBA sous le nom IRGANOX 1520; le 4,6-bis(dodécylthiométhyl)o-crésol notamment celui commercialisé par CIBA sous le nom IRGANOX 1726.

Les polycondensats selon l'invention peuvent être utilisés très avantageusement dans une composition notamment cosmétique ou pharmaceutique, qui comprend par ailleurs un milieu physiologiquement, notamment cosmétiquement ou pharmaceutiquement, acceptable, c'est-à-dire un milieu compatible avec les tissus cutanés comme la peau du visage ou du corps, et les matières kératiniques telles que les cheveux, les cils, les sourcils et les ongles.

La quantité de polycondensat présente dans les compositions dépend bien entendu du type de composition et des propriétés recherchées et peut varier à l'intérieur d'une gamme très large, comprise généralement entre 0,1 et 70% en poids, de préférence entre 1 et 50% en poids, notamment entre 10 et 45% en poids, voire entre 20 et 40% en poids, et mieux entre 25 et 35% en poids, par rapport au poids de la composition cosmétique ou pharmaceutique finale.

La composition peut alors comprendre, selon l'application envisagée, les constituants habituels à ce type de composition.

La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
   Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
   Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.
   De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
   On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810^{®}", "812^{®} et "818^{®} " par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
   Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

Préférentiellement, le milieu physiologiquement acceptable de la composition selon l'invention comprend, dans une phase grasse liquide, au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15 et/ou la D5 (décaméthylcyclopentasiloxane).

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en C₆ à C₄₀, notamment en C10-C40; les éthers de propylène glycol liquides à température ambiante tels que le monométhyléther de propylène glycol, l'acétate de monométhyléther de propylène glycol, le mono n-butyl éther de dipropylène glycol;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone;
- les aldéhydes liquides à température ambiante tels que le benzaldéhyde, l'acétaldéhyde;

La phase grasse liquide peut représenter 1 à 90% en poids de la composition, notamment de 5 à 75% en poids, en particulier de 10 à 60% en poids, voire de 25 à 55% en poids, du poids total de la composition.

La composition selon l'invention peut comprendre avantageusement un agent épaississant qui peut en particulier être choisi parmi :
- les silices notamment hydrophobes, telles que celles décrites dans le document EP-A-898960, et par exemple commercialisées sous les références "AEROSIL R812^{®}" par la société Degussa, "CAB-O-SIL TS-530^{®}", "CAB-O-SIL TS-610^{®}", "CAB-O-SIL TS-720^{®}"par la société Cabot, "AEROSIL R972^{®}", "AEROSIL R974^{®}" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La quantité d'agent épaississant dans la composition selon l'invention peut aller de 0,05 à 40% en poids, par rapport au poids total de la composition, de préférence de 0,5 à 20% et mieux de 1 à 15% en poids.

La composition selon l'invention peut également comprendre au moins une cire d'origine végétale, animale, minérale ou de synthèse, voire siliconée.

On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La quantité de cire dans la composition selon l'invention peut aller de 0,1 à 70% en poids, par rapport au poids total de la composition, de préférence de 1 à 40% en poids, et mieux de 5 à 30% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.

Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.

Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les anti-mousses, les neutralisants, les stabilisants, les polymères et notamment les polymères filmogènes liposolubles, et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.

Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention présentant une brillance et une tenue de ladite brillance améliorées par rapport à l'état de l'art, elles peuvent être utilisées pour le soin ou le maquillage des matières kératiniques telles que les cheveux, la peau, les cils, les sourcils, les ongles, les lèvres, le cuir chevelu et plus particulièrement pour le maquillage des lèvres, des cils et/ou du visage.

Elles peuvent donc se présenter sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux; elles se présentent avantageusement sous forme de mascara, de rouge à lèvres, de brillant à lèvres (gloss), de fard à joues ou à paupières, de fond de teint.

L'invention a encore pour objet un procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie précédemment.

Ce procédé selon l'invention permet notamment le soin ou le maquillage des lèvres, par application d'une composition de rouge à lèvres ou de brillant à lèvres (gloss) selon l'invention.

L'invention est illustrée plus en détail dans les exemples suivants.

### Méthode de mesure de la viscosité

La viscosité à 80°C ou à 110°C du polymère est mesurée à l'aide d'un viscosimètre à cône plan de type BROOKFIELD CAP 1000+.

Le cône-plan adapté est déterminé par l'homme du métier, sur la base de ses connaissances; notamment :
- entre 50 et 500 mPa.s, on peut utiliser un cône 02
- entre 500 et 1000 mPa.s : cône 03
- entre 1000 et 4000 mPa.s : cône 05
- entre 4000 et 10000 mPa.s : cône 06

### Exemple 1: Synthèse du pentaérythrityl benzoate/isophtalate/isostéarate/ stéarate

Dans un réacteur équipé d'une agitation mécanique, d'une arrivée d'argon et d'un système de distillation, on charge 20 g d'acide benzoïque, 210 g d'acide stéarique, 70 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures. On obtient ainsi 450 g de polycondensat pentaérythrityl benzoate/isophtalate/isostéarate /stéarate sous la forme d'une huile très épaisse.

Le polycondensat présente les caractéristiques suivantes:
- soluble à 50% en poids, à 70°C, dans le Parléam
- Indice d'acide = 7,1
- η_{110°C} = 850 mPa.s
- Mw = 28500
- rapport entre le nombre de mole d'acide monocarboxylique aromatique et le nombre de mole d'acides monocarboxyliques non aromatiques : 0,166.

### Exemple 2 : Synthèse du pentaérythrityl béhénate/benzoate/isophtalate /isostéarate

Dans un réacteur équipé d'une agitation mécanique, d'une arrivée d'argon et d'un système de distillation, on charge 20 g d'acide benzoïque, 140 g d'acide béhénique, 140 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.

On obtient ainsi 440 g de polycondensat pentaérythrityl béhénate/benzoate/ isophtalate/isostéarate sous la forme d'une huile très épaisse.

Le polycondensat présente les caractéristiques suivantes :
- soluble à 50% en poids, à 70°C, dans le Parléam
- Indice d'acide = 4,2
- η_{110°C} = 2050 mPa.s
- rapport entre le nombre de mole d'acide monocarboxylique aromatique et le nombre de mole d'acides monocarboxyliques non aromatiques : 0,181.

### Exemple 3

De manière similaire aux exemples précédents, on prépare les polycondensats suivants (les % sont en poids):

| | Polyol (% et nature) | Acide aromatique (% et nature) | Acide polycarboxylique ou anhydride (% et nature) | Acides non aromatiques (% et nature) | Solubilité * |
|---|---|---|---|---|---|
| Exemple A | 20,4% Pentaérythritol | 4,1% benzoïque | 18,3% Acide isophtalique | 28,6% isostéarique + 14,3% isononanoïque + 14,3% stéarique | à 25°C |
| Exemple B | 20% Pentaérythritol | 4% benzoïque | 20% Acide isophtalique | 18% isostéarique + 38% stéarique | à 25°C |
| Exemple C | 20% Pentaérythritol | 4% benzoïque | 20% Acide isophtalique | 28% isostéarique + 28% stéarique | à 25°C |
| Exemple D | 19,8% Pentaérythritol | 4% benzoïque | 19,8% Acide isophtalique | 40,6% isostéarique + 15,8% stéarique | à 25°C |
| Exemple E | 19,8% Pentaérythritol | 4% benzoïque | 19,8% acide isophtalique | 48,5% isostéarique + 7,9% stéarique | à 25°C |
| Exemple F | 19,8% Pentaérythritol | 4% benzoïque | 19,8% acide isophtalique | 52,4% isostéarique + 4% stéarique | à 25°C |
| Exemple G | 25,5% Diglycérol | 3,9% benzoïque | 15,7% acide sébacique | 34,9% isostéarique + 20% laurique | à 25°C |
| Exemple H | 25% triméthylol-propane | 2,1% m-toluique | 14,6% anhydride phtalique | 18,3% isostéarique + 40% behenique | à 70°C |
| Exemple I | 21,9% érythritol | 6,3% terbutyl-benzoique | 13,5% acide sébacique | 8,3% isooctanoïque + 50% stéarique | à 70°C |
| Exemple J | 20,7% glycérol | 8,5% terbutyl-benzoique | 15,9% acide adipique | 45,9% isononanoïque + 9% béhénique | à 25°C |
| Exemple K | 20% Pentaérythritol | 4% benzoïque | 20% acide isophtalique | 28% isostéarique + 28% béhénique | à 70°C |

| | | | | | |
|---|---|---|---|---|---|
| * 'à 25°C' indique que le polymère est soluble à 50% en poids, à 25°C, dans le Parléam; 'à 70°C' indique que le polymère est soluble à 50% en poids, à 70°C, dans le Parléam. | | | | | |

### Exemple 4

On a préparé un rouge à lèvres ayant la composition suivante:
- polycondensat de l'exemple C 30 g
- cire de polyéthylène 11 g
- pigments et charges 7 g
- Parléam (isoparaffine hydrogénée) qsp 100 g

### Exemple 5

On a préparé un rouge à lèvres ayant la composition suivante :
- polycondensat de l'exemple D 30 g
- cire de polyéthylène 11 g
- pigments et charges 7 g
- Parléam (isoparaffine hydrogénée) qsp 100 g

### Exemple 6

On prépare un rouge à lèvres ayant la composition suivante :
- polycondensat de l'exemple 1 10 g
- cire de polyéthylène 11 g
- pigments et charges 7 g
- Parléam (isoparaffine hydrogénée) qsp 100 g

## Revendications

1. Composition cosmétique ou pharmaceutique comprenant, dans un milieu cosmétiquement ou pharmaceutiquement acceptable, au moins un polycondensat susceptible d'être obtenu par réaction :
- de 10 à 30% en poids, par rapport au poids total du polycondensat, d'au moins un polyol comprenant 3 à 6 groupes hydroxyles;
- de 22 à 80% en poids, par rapport au poids total du polycondensat, d'au moins un acide monocarboxylique non aromatique, saturé ou insaturé, linéaire, ramifié et/ou cyclique, comprenant 10 à 32 atomes de carbone, et ayant une température de fusion supérieure ou égale à 25°C;
- de 0,1 à 35% en poids, par rapport au poids total du polycondensat, d'au moins un acide monocarboxylique non aromatique, saturé ou insaturé, linéaire, ramifié et/ou cyclique, comprenant 6 à 32 atomes de carbone, et ayant une température de fusion strictement inférieure à 25°C;
- de 0,1 à 10 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 1 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, pouvant comprendre des hétéroatomes; et/ou un anhydride cyclique d'un tel acide polycarboxylique; et/ou une lactone comprenant au moins un groupement COOH.

2. Composition selon la revendication 1, dans laquelle le polyol est choisi parmi, seul ou en mélange :
- les triols, tels que 1,2,4-butanetriol, le 1,2,6-hexanetriol, le triméthyloléthane, le triméthylolpropane, le glycérol;
- les tétraols, tels que le pentaérythritol, l'érythritol, le diglycérol ou le ditriméthylolpropane;
- les pentols tels que le xylitol,
- les hexols tels que le sorbitol et le mannitol; ou encore le dipentaérythritol ou le triglycérol.

3. Composition selon l'une des revendications précédentes, dans laquelle le polyol, ou le mélange de polyols, représente 12 à 25% en poids, et mieux 14 à 22% en poids, du poids total du polycondensat.

4. Composition selon l'une des revendications précédentes, dans laquelle l'acide monocarboxylique non aromatique ayant une température de fusion supérieure ou égale à 25°C est choisi parmi, seul ou en mélange:
- les acides monocarboxyliques saturés tels que l'acide décanoïque, l'acide laurique, l'acide tridécanoïque, l'acide myristique, l'acide palmitique, l'acide stéarique, l'acide arachidique, l'acide béhénique, l'acide cérotique (hexacosanoïque);
- les acides monocarboxyliques insaturés mais non aromatiques, tels que l'acide pétrosélinique, l'acide vaccénique, l'acide élaidique, l'acide gondoïque, l'acide gadoléique, l'acide érucique, l'acide nervonique.

5. Composition selon l'une des revendications précédentes, dans laquelle l'acide monocarboxylique non aromatique ayant une température de fusion supérieure ou égale à 25°C, ou le mélange desdits acides, représente 25 à 75% en poids, voire 27 à 70% en poids, et mieux 28 à 65% en poids, du poids total du polycondensat.

6. Composition selon l'une des revendications précédentes, dans laquelle l'acide monocarboxylique non aromatique ayant une température de fusion inférieure à 25°C, est choisi parmi, seul ou en mélange:
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide isononanoïque, l'acide isostéarique;
- les acides monocarboxyliques insaturés mais non aromatiques, tels que l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide linoléique, l'acide linolénique, l'acide arachidonique.

7. Composition selon l'une des revendications précédentes, dans laquelle l'acide monocarboxylique non aromatique ayant une température de fusion inférieure à 25°C, ou le mélange desdits acides, représente 0,5 à 32% en poids, voire 1 à 30% en poids, et mieux 2 à 28% en poids, du poids total du polycondensat.

8. Composition selon l'une des revendications précédentes, dans laquelle l'acide monocarboxylique aromatique est choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque.

9. Composition selon l'une des revendications précédentes, dans laquelle l'acide monocarboxylique aromatique, ou le mélange desdits acides, représente 0,5 à 9,95% en poids, notamment 1 à 9,5% en poids, et mieux 1,5 à 8% en poids, du poids total du polycondensat.

10. Composition selon l'une des revendications précédentes, dans laquelle l'acide polycarboxylique, son anhydride ou la lactone, est choisi parmi, seul ou en mélange,
- les acides polycarboxyliques linéaires, ramifiés et/ou cycliques, saturés ou insaturés, voire aromatiques, comprenant 2 à 50 atomes de carbone; ledit acide comprenant au moins deux groupes carboxyliques COOH; et pouvant optionnellement comprendre 1 à 10 hétéroatomes, identiques ou différents, choisis parmi O, N et S; et/ou pouvant optionnellement comprendre au moins un radical perfluoré choisi parmi -CF₂- (divalent) ou -CF₃; et leurs anhydrides;
- les acides polycarboxyliques à chaîne saturée ou insaturée, linéaire ou ramifiée, comprenant au moins un hétéroatome choisi parmi O, N et/ou S, notamment 1 à 10 hétéroatomes identiques ou différents, et/ou comprenant au moins un radical perfluoré -CF₂- ou -CF₃ et possédant par ailleurs au moins 2 groupes carboxyliques COOH; et leurs anhydrides;
- les acides polycarboxyliques hétérocycliques, saturé ou insaturé, voire aromatique, comprenant au moins un hétéroatome choisi parmi 0, N et/ou S, notamment 1 à 10 hétéroatomes identiques ou différents, et au moins 2 groupes carboxyliques COOH; et leurs anhydrides;
- les acides polycarboxyliques dérivés de sucre, susceptibles d'être obtenus notamment par oxydation d'un aldose, et comprenant au moins 2 groupes carboxyliques COOH; et leurs anhydrides;
- l'anhydride itaconique et le 1,4-monoanhydride de l'acide 1,4,5,8-naphthalenetetracarboxylique;
- les aminoacides polycarboxyliques (y compris hétérocycliques), c'est-à-dire les acides polycarboxyliques à chaîne saturée ou insaturée, linéaire, ramifiée, et/ou cyclique, comprenant éventuellement au moins un hétéroatome choisi parmi O, N et/ou S, notamment 1 à 10 hétéroatomes identiques ou différents, et/ou comprenant éventuellement au moins un radical perfluoré -CF₂- ou -CF₃, et comprenant en outre au moins une fonction amine primaire, secondaire ou tertiaire et possédant par ailleurs au moins 2 groupes carboxyliques COOH; et leurs anhydrides;
- les lactones comprenant 1, 2 ou 3 groupements COOH, et 5 à 14 atomes de carbone, notamment 6 à 13, voire 6 à 12 atomes de carbone.

11. Composition selon l'une des revendications précédentes, dans laquelle l'acide polycarboxylique et/ou son anhydride cyclique et/ou la lactone représente 5 à 35% en poids, notamment 10 à 30% en poids, et mieux 14 à 25% en poids, du poids total du polycondensat final.

12. Composition selon l'une des revendications précédentes, dans laquelle le polycondensat est présent en une quantité comprise entre 0,1 et 70% en poids, de préférence entre 1 et 50% en poids, notamment entre 10 et 45% en poids, voire entre 20 et 40% en poids, et mieux entre 25 et 35% en poids, par rapport au poids de la composition cosmétique ou pharmaceutique finale.

13. Composition selon l'une des revendications précédentes, se présentant sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux.

14. Procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie à l'une des revendications 1 à 13.

15. Polycondensat susceptible d'être obtenu par réaction :
- de 10 à 30% en poids, par rapport au poids total du polycondensat, d'au moins un polyol comprenant 3 à 6 groupes hydroxyles;
- de 22 à 80% en poids, par rapport au poids total du polycondensat, d'au moins un acide monocarboxylique non aromatique, saturé ou insaturé, linéaire, ramifié et/ou cyclique, comprenant 10 à 32 atomes de carbone, et ayant une température de fusion supérieure ou égale à 25°C;
- de 0,1 à 35% en poids, par rapport au poids total du polycondensat, d'au moins un acide monocarboxylique non aromatique, saturé ou insaturé, linéaire, ramifié et/ou cyclique, comprenant 6 à 32 atomes de carbone, et ayant une température de fusion strictement inférieure à 25°C;
- de 0,1 à 10% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 1 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, pouvant comprendre des hétéroatomes; et/ou un anhydride cyclique d'un tel acide polycarboxylique; et/ou une lactone comprenant au moins un groupement COOH.

16. Procédé de préparation des polycondensats selon la revendication 15, consistant :
- à mélanger le polyol et les acides monocarboxyliques aromatiques et non aromatiques,
- à chauffer le mélange sous atmosphère inerte, d'abord jusqu'à la température de fusion, et ensuite à une température comprise entre 150 et 220°C jusqu'à consommation complète des acides monocarboxyliques, puis
- à éventuellement refroidir le mélange à une température comprise entre 90 et 150°C,
- à ajouter l'acide polycarboxylique et/ou l'anhydride cyclique et/ou la lactone, puis
- à chauffer à nouveau à une température inférieure ou égale à 220°C.
